# EUROPEAN PATENT SPECIFICATION

(11) **EP 4 326 248 B1**
(45) Date of publication and mention of the grant of the patent: **01.04.2026**
(21) Application number: 22724768.1
(22) Date of filing: 24.04.2022
(51) Int. Cl.: A61K 31/20, A61K 31/4412, A61K 31/513, A61K 45/06, A61K 39/00, A61K 39/395, A61P 35/00, C07K 16/28, G01N 33/00

(54) **3-HYDROXYDODECANOIC ACID FOR USE IN THE TREATMENT OF CANCER**
3-HYDROXYDODECANSÄURE ZUR BEHANDLUNG VON KREBS
ACIDE 3-HYDROXYDODECANOÏQUE POUR SON UTILISATION DANS LE TRAITEMENT DU CANCER

(30) Priority: 23.04.2021 EP 21170194
(43) Date of publication of application: 28.02.2024
(73) Proprietor: Universität Zürich, 8006 Zürich (CH)
(72) Inventor: SCHARL, Michael, 8904 Aesch (CH); MONTALBAN-ARQUES, Ana, 8091 Zürich (CH); KATKEVICIUTE, Egle, 8091 Zürich (CH); MORSY, Yasser, 8091 Zürich (CH)
(74) Representative: Schulz Junghans Patentanwälte PartGmbB
(86) International application number: PCT/EP2022/060793
(87) International publication number: WO 2022/223831

(56) References cited:
- EP-A1- 3 372 590
- WO-A1-2015/120100
- WO-A1-2022/096573
- PETERS ANNA ET AL: "Hydroxycarboxylic acid receptor 3 and GPR84 - Two metabolite-sensing G protein-coupled receptors with opposing functions in innate immune cells", PHARMACOLOGICAL RESEARCH, vol. 176, 27 December 2021 (2021-12-27), AMSTERDAM, NL, pages 106047, XP055978492, ISSN: 1043-6618, DOI: 10.1016/j.phrs.2021.106047
- LIU YANG ET AL: "Design and Synthesis of 2-Alkylpyrimidine-4,6-diol and 6-Alkylpyridine-2,4-diol as Potent GPR84 Agonists", ACS MEDICINAL CHEMISTRY LETTERS, vol. 7, no. 6, 9 June 2016 (2016-06-09), US, pages 579 - 583, XP055861093, ISSN: 1948-5875, DOI: 10.1021/acsmedchemlett.6b00025
- PILLAIYAR THANIGAIMALAI ET AL: "6-(Ar)Alkylamino-Substituted Uracil Derivatives: Lipid Mimetics with Potent Activity at the Orphan G Protein-Coupled Receptor 84 (GPR84)", ACS OMEGA, vol. 3, no. 3, 31 March 2018 (2018-03-31), US, pages 3365 - 3383, XP055861353, ISSN: 2470-1343, DOI: 10.1021/acsomega.7b02092

## Description

The present invention relates to 2-hydroxy-dodecanoic acid for use in the treatment or prevention of recurrence of particular cancer types.

### Background of the Invention

Checkpoint inhibitor antibodies can lead to durable protective immune responses to cancer, yet a majority of patients fail to respond to immunotherapy treatment regimes. For example, anti-PD1, anti-PDL1, and/or anti-CTLA4 monoclonal antibodies are effective in at most one half of melanoma patients, and only about 4-5% of metastatic colorectal cancer (CRC) patients characterised by high microsatellite instability. In addition, these drugs can induce severe, and occasionally life-threatening side-effects.

The gut microbiome has recently emerged as a modulator of immunotherapy and chemotherapeutic agents by means of activation of anti-tumour responses. A dysbiotic gut microbiota characterised by a reduction of *Clostridiales* bacteria (encompassing butyrate-producing species) has been correlated with increased incidence of CRC and other diseases. However, there are at present few approaches which can ameliorate the harmful effects of an unbalanced gut bacteria. Interventions such as antibiotics, prebiotics, probiotics and faecal transplants have been used to address this problem, but each has particular limitations, such as regulatory challenges for live bacterial preparations, and possible side-effects.

Aside from the use of BCG to treat certain types of bladder cancer, the current guidelines for therapeutic or preventative administration live biotherapeutic products, particularly bacterial consortia are unclear. In addition, consortia may be more difficult to manufacture on an industrial scale compared to a single strain of microbe as a medicament.

WO 2022/096573 A1 discloses 3R-hydroxydodecanoic acid used to treat a skin condition.

WO 2015/120100 A1 discloses to 3R-hydroxydodecanoic acid used to preserve food and sanitise surfaces.

Based on the above-mentioned state of the art, the objective of the present invention is to provide improved compositions for use in the treatment of cancer. This objective is attained by the subject-matter of the independent claims of the present specification.

### Summary of the Invention

The subject of this invention is based on the characterization of commensal bacterial-derived metabolites which mediate the observed therapeutic effects of enteral commensal bacteria delivered as an antineoplastic therapy.

In general, wherever reference is made herein to a hydroxycarboxylic acid for use in medicine or in treatment of a specific condition, this mention implies the use of a pharmaceutically acceptable salt wherein the carboxylic acid group is in anion form paired by a suitable cation. A first aspect of the invention relates to a hydroxycarboxylic acid of the formula:

(I) CH₃(CH₂)₈CHOH-(CH₂)-CO₂H

as the protonated acid, or in the form of a pharmaceutically acceptable salt, for use in the treatment or prevention, including prevention of recurrence, of cancer, for example colorectal cancer. The composition may be provided for treatment or prevention of a condition that is expected to benefit from an activation of the patient's CD8 T cells.

The invention further relates to a combination medicament for use in the treatment or the prevention of recurrence of cancer comprising the hydroxycarboxylic acid according to Formula (I), and an antineoplastic treatment.

### Terms and definitions

For purposes of interpreting this specification, the following definitions will apply and whenever appropriate, terms used in the singular will also include the plural, and vice versa. In the event that any definition set forth below conflicts with any document given herein as reference, the definition set forth shall control.

Where a range of values is provided, it is understood that each intervening value, to the tenth of the unit of the lower limit, unless the context clearly dictate otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a," "or," and "the" include plural referents unless the context clearly dictates otherwise.

Unless defined otherwise, all technical and scientific terms used herein have the same meaning as commonly understood by one of ordinary skill in the art (e.g., in cell culture, molecular genetics, nucleic acid chemistry, hybridization techniques and biochemistry). Standard techniques are used for molecular, genetic and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods. The stereochemical configuration of compounds follows the rules established by Cahn, Ingold and Prelog (see March, Jerry; Michael B., Smith (2007). March's advanced organic chemistry: reactions, mechanisms, and structure (6. ed.). Hoboken, NJ: Wiley-Interscience. pp. 155-162. ISBN 978-0-471-72091-1)

The term *patient* in the context of the present specification relates to a human subject.

In the context of the present specification, the term *checkpoint inhibitory agent* or *checkpoint inhibitor antibody* is meant to encompass a cancer immunotherapy agent, particularly an antibody (or antibody-like molecule) capable of disrupting an inhibitory signalling cascade that limits immune cell activation, known in the art as an immune checkpoint mechanism. The terms *checkpoint inhibitory agent* or *checkpoint inhibitor antibody* include, without being limited to, an antibody to CTLA-4 (Uniprot P16410), PD-1 (Uniprot Q15116), PD-L1 (Uniprot Q9NZQ7), B7H3 (CD276; Uniprot Q5ZPR3), VISTA (Uniprot Q9H7M9), TIGIT (UniprotQ495A1), TIM-3 (HAVCR2, Uniprot Q8TDQ0), CD158 (killer cell immunoglobulin-like receptor family), and/or TGF-beta (P01137).

The term cancer immunotherapy agent encompasses, without being limited to, the clinically available antibody drugs ipilimumab (Bristol-Myers Squibb; CAS No. 477202-00-9), nivolumab (Bristol-Myers Squibb; CAS No 946414-94-4), pembrolizumab (Merck Inc.; CAS No. 1374853-91-4), pidilizumab (CAS No. 1036730-42-3), atezolizumab (Roche AG; CAS No. 1380723-44-3), avelumab (Merck KGaA; CAS No. 1537032-82-8), durvalumab (Astra Zeneca, CAS No. 1428935-60-7), and/or cemiplimab (Sanofi Aventis; CAS No. 1801342-60-8).

As used herein, the term *pharmaceutical formulation* refers to a preparation of a hydroxydicarboxylic acid, or an alpha-beta unsaturated dicarboxylic acid, or a synthetic GPR84 agonist, optionally together with a pharmaceutically acceptable carrier. As used herein, the term *pharmaceutically acceptable carrier* includes any solvents, dispersion media, coatings, surfactants, antioxidants, preservatives (for example, antibacterial agents, antifungal agents), isotonic agents, absorption delaying agents, salts, preservatives, drugs, drug stabilizers, binders, excipients, disintegration agents, lubricants, sweetening agents, flavouring agents, dyes, and the like and combinations thereof, as would be known to those skilled in the art (see, for example, Remington: the Science and Practice of Pharmacy, ISBN 0857110624).

The composition can be in various forms including, but not limited to, granules, powders, emulsions, suspensions, solutions, gels, dermal absorption systems, capsules or tablets. The hydroxycarboxylic acid, GPR84 agonist, or its salt, can be included in a food product, which may optionally include other nutrients or prebiotics such as dietary fibre.

As used herein, the term *treating* or *treatment* of any disease or disorder (e.g. cancer) refers in one embodiment, to ameliorating the disease or disorder (e.g. slowing or arresting or reducing the development of the disease or at least one of the clinical symptoms thereof). In another embodiment "treating" or "treatment" refers to alleviating or ameliorating at least one physical parameter including those which may not be discernible by the patient. In yet another embodiment, "treating" or "treatment" refers to modulating the disease or disorder, either physically, (e.g., stabilization of a discernible symptom), pharmaceutically, (e.g., stabilization of a physical parameter), or both. Methods for assessing treatment and/or prevention of disease are generally known in the art, unless specifically described herein below. Any references in the description to methods of treatment, diagnosis or surgery refer to the compounds, pharmaceutical compositions and medicaments of the present invention for use in a method for treatment, diagnosis or surgery of the human or animal body by therapy.

### Detailed Description of the Invention

A first aspect of the invention relates to a *hydroxycarboxylic acid* described by the formula CH₃(CH₂)₈ CHOH-(CH₂)-CO₂H (I): 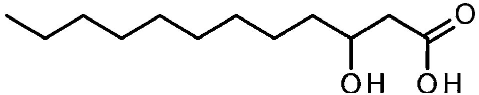
(I) 3-hydroxydodecanoic acid (CAS no. 1883-13-3, Pubchem 94216) or a pharmaceutically acceptable salt thereof, for use in the treatment or prevention, including prevention of recurrence, of cancer.

It is understood that in a physiological context, the carboxylic acid described herein is at least partially disassociated into the respective anion or dianion and a corresponding cation. Depending on the type of formulation and the route of administration chosen, the carboxylic acid described herein may be provided as a pharmaceutically acceptable salt.

Pharmaceutically acceptable salts comprise the ionized drug and an oppositely charged counterion. Non-limiting examples of pharmaceutically acceptable cationic salt forms include aluminium, benzathine, calcium, ethylene diamine, lysine, magnesium, meglumine, potassium, procaine, sodium, tromethamine and zinc.

3-hydroxydodecanoic acid, also known as 3-hydroxylauric acid, is a medium chain fatty acid derivative of a 3-hydroxydodecanedioic acid identified in metabolic screening of the serum of mice with CRC treated with a mix of *Clostridiales* bacteria to ameliorate disease. The data presented in the examples show that the compound having the formula CH₃(CH₂)₈ CHOH-(CH₂)-CO₂H can inhibit tumour growth when administered orally *in vivo,* in various pre-clinical mouse models of solid tumours.

Systematic testing of which derivatives of 3-hydroxydicarboxylic acid derivatives with differing hydroxyl positions, carboxyl groups, and carbon chain length were associated with the desired immune-stimulatory effects identified the compound of this formula as the preferred agent for inhibiting tumour growth.

In certain embodiments, the hydroxycarboxylic acid for use according to the invention is characterized by a carbon bearing the hydroxyl group in the S configuration. The S configuration of formula CH₃(CH₂)₈ CHOH-(CH₂)-CO₂H is shown in formula I-S:

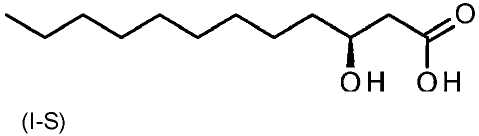

In certain embodiments, the hydroxycarboxylic acid for use according to the invention is characterized by a carbon bearing the hydroxyl group in the R configuration. The R configuration of formula CH₃(CH₂)₈ CHOH-(CH₂)-CO₂H is shown in formula I-R:

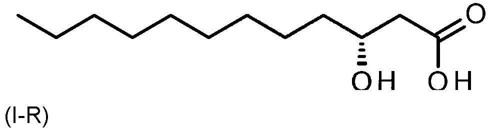

A second aspect of the invention relates to a pharmaceutical formulation of hydroxycarboxylic acid as specified by formula I, or a pharmaceutically acceptable salt thereof, formulated for enteral, topical, intratumoural, intravenous and/or parenteral administration, for use in the treatment or prevention, including prevention of recurrence, of cancer. In particular embodiments, the formulation is for use in treating a solid tumour form of cancer. A non-exclusive list of epithelial cell cancers or other solid cancers which might benefit from treatment with the compounds or formulations for use according to the invention includes lung, breast, brain, prostate, spleen, pancreatic, biliary tract, cervical, ovarian, head and neck, oesophageal, gastric, liver, skin, kidney, bone, testicular, small intestinal, bladder, skin cancer, melanoma or sarcoma. In more particular embodiments, the hydroxycarboxylic acid formulation is for use treating colorectal cancer.

In certain other embodiments, the hydroxycarboxylic acid as described herein, or a pharmaceutically acceptable salt thereof, is for use in the treatment of a patient diagnosed with lung cancer.

In certain other embodiments, the hydroxycarboxylic acid as described herein, or a pharmaceutically acceptable salt thereof, is for use in the treatment of a patient diagnosed with colorectal cancer (CRC), which is understood to encompass non-dysplastic serrated polyps, or serrated crypt foci. In some embodiments, the CRC has a CpG island methylator phenotype (CIMP). In some embodiments, the CRC is a serrated neoplasia.

In certain other embodiments, the hydroxycarboxylic acid as described herein, or a pharmaceutically acceptable salt thereof, is for use in the treatment of a patient diagnosed with a non-epithelial cell derived cancer, particularly melanoma.

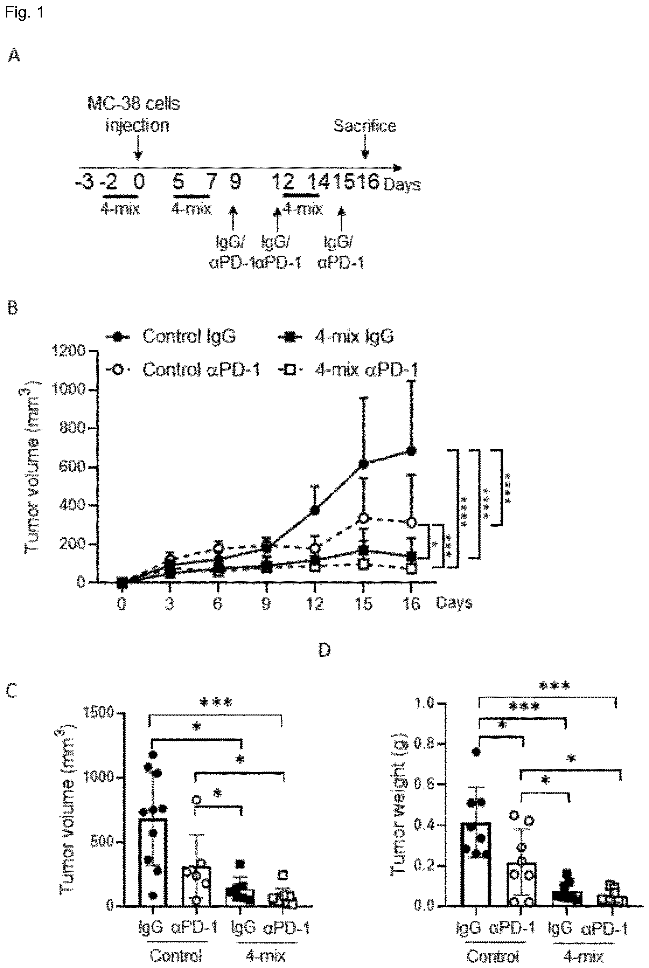

Evidence presented in the examples demonstrates parenterally administered 3-hydroxydodecanoic acid is an effective treatment for pre-clinical CRC cancer models of colorectal cancer, melanoma, and lung cancer. The skilled artisan will recognise that this approach can likely be applied to other solid or epithelial cell-derived cancer types, as 3-hydroxydodecanoic acid administration is equivalent to the therapeutic oral administration of the 4-mix of *Clostridiales* from which the metabolite was identified, wherein administration of a clostridia consortia comprising the four *Clostridiales* strains *Eubacterium hallii, Faecalibacterium prausnitzii, Roseburia intestinalis,* and *Anaerostipes caccae* reduced tumour growth in pre-clinical models of colorectal cancer, melanoma, breast cancer and lung cancer (Fig. 1-3).

"Prevention of cancer" includes and may be particularly important for, prevention of recurrence of cancer after remission, as a consequence of treatment. The hydroxydodecanoic can increase, or induce an immune response to cancer. This is reasonably expected to have a positive effect on the clinical outcome of the subject if the cancer is a solid, malignant tumour characterized by a paucity of tumour infiltrating immune cytotoxic CD8+ T cells. These immune cells are thought to be those most important for killing tumour cells and thus inhibiting tumour growth and spread.

In certain embodiments the 3-hydroxydodecanoic acid is provided for use in a patient whose tumour has been determined to be characterised by a paucity of infiltrating immune cells, such as natural killer (NK) cells, NKT cells, or T cells, particularly cytotoxic CD8+ T cells. A paucity, or absence of immune infiltration may be identified by means known in the art, including, but not limited to immunohistochemical staining with immune markers such as CD3, or CD45, and may encompass samples where immune infiltrate is peritumoural, rather than within tumour tissues (Hendry 2017 Adv. Anat. Pathol. 24(6):311). Low numbers of immune infiltration may be defined, for example, as under 1000, or under 500 CD3 positive cells counted on a tumour standard microarray slide section, as determined by histochemical staining of CD3 with reference to an isotype control. In certain embodiments, the bacterial composition is provided for use in such subject in order to increase or induce an immune response to the cancer. In other words, the bacterial composition is provided for use in a patient whose tumour has been determined to by characterised by no, or limited, immune cell infiltration. These immune cells are thought to be those most important for killing tumours cells and thus inhibiting tumour growth and spread.

A further set of embodiments of the invention refer to 3-hydroxydodecanoic acid, or any of its pharmaceutically acceptable salt forms, as part of a combination medicament for use in the treatment or the prevention of recurrence of cancer.

Due to the low toxicity of the metabolic intervention, it is envisioned that the 3-hydroxydodecanoic acid may be administered at the same time, or overlapping with another antineoplastic pharmaceutical drug.

In particular embodiments, the 3-hydroxydodecanoic acid may be delivered as a component of a combination medicament, additionally comprising a cancer immunotherapy. In particular embodiments, the metabolite is delivered alongside a checkpoint inhibitor antibody selected from anti-PD-1, anti-PD-L1, anti-PD-L2, or anti-CTLA-4.

In particular embodiments, the 3-hydroxydodecanoic is administered with an anti-PD1 antibody.

The data in the examples show that treatment with 3-hydroxydodecanoic acid in combination with an anti-PD1 antibody, can provide better protection against cancer than anti-PD1 antibody treatment alone. PD-L1, or PD-L2 blocking antibodies are expected to have a similar effect.

In other embodiments, the 3-hydroxydodecanoic acid for treatment or prevention of recurrence of cancer is administered without previous, concurrent, or subsequent administration of an antineoplastic pharmaceutical drug. In other words, the subject receives the hydroxycarboxylic acid, without any other additional preventative or therapeutic drug treatment for cancer. Therapeutic treatment in this sense is understood to encompass checkpoint inhibitory agents, particularly checkpoint inhibitor antibodies, as well as antineoplastic chemotherapeutic agents, for example the 5-fluorouracil chemotherapy drug tested in the examples.

The data in the examples show that treatment with 3-hydroxydodecanoic acid according to the invention can offer equal inhibition of cancer compared to standard-of-care 5-fluorouracil (5-FU) treatment, and additional protection combined with an anti-PD1 immunotherapy. Both PD-1 antibodies and 5-FU have been associated with considerable side effects in the clinic. The inventors identified 3-hydroxydodecanoic acid for use in treating a model of colon cancer, and predict this molecule may replace *Clostridiales* treatment to enhance immune responses against cancer.

### Routes of Administration and Dosage

The specific therapeutically effective dose level of the hydroxycarboxylic acid for use according to the invention for any particular subject will depend upon a variety of factors, including the presence or absence of cancer, the type of cancer being treated, the severity of the cancer, the rate of clearance or elimination from circulation, the route of administration, the duration of treatment, the drugs (if any) used in combination with the organism, the age, body weight, sex, diet, and general health of the subject, and similar factors well known in the medical arts and sciences. In certain embodiments, the hydroxycarboxylic acid for use according to the invention, or as a component of combination treatment, can be in unit dosage of about 1-1000 mg of active ingredient(s) for a subject of about 50-70 kg. In certain embodiments, the compounds of the invention may be administered in a single daily dose, or the total daily dosage may be administered in divided doses of two, three, or four times daily.

The data in the examples show that several doses of CH₃(CH₂)₃ CHOH-(CH₂)-CO₂H or the compound 2-(hexylthio)pyramidine-4,6-diol (reference) inhibited tumour growth in a murine model of CRC. when a 200ul dose was administered 3 times, each 200ul volume containing the active compound at concentrations of 200µM. A therapeutically effective human dosage level of a compound for use according to the invention can be ascertained by the skilled artisan by means of this information based on animal models of disease, extrapolated with reference to relative mouse to human body surface area measurements (Nair A. B and Jacob S. J. Basic Clin. Pharm. 2016 7(2):27-31), or by reference to the dosages of metabolites or agonist delivered in human clinical trails through similar routes of administration.

In certain particular embodiments, the 3-hydroxydodecanoic acid for use according to the invention is formulated for enteral, topical, intra-tumoural, intravenous or parental administration. Dosage forms may be for enteral administration, such as nasal, buccal, rectal, transdermal or oral administration, or as an inhalation form or suppository. Alternatively, parenteral administration may be used, such as subcutaneous, intravenous, intrahepatic or intramuscular injection forms. Optionally, a pharmaceutically acceptable carrier and/or excipient may be present.

In embodiments of the invention relating to topical uses of the compounds of the invention, the pharmaceutical composition is formulated in a way that is suitable for topical administration such as aqueous solutions, suspensions, ointments, creams, gels or sprayable formulations, e.g., for delivery by aerosol or the like, comprising the active ingredient together with one or more of solubilizers, stabilizers, tonicity enhancing agents, buffers and preservatives that are known to those skilled in the art.

The term *"topical"* is understood to mean local administration, in other words applied directly to a tumour tissue. This could be in the form a cream or gel applied to an accessible tumour such as a form of skin cancer, or it could also apply to using an injection to deliver the 3-hydroxydodecanoic acid in solution directly into a solid tumour.

In certain embodiments, the 3-hydroxydodecanoic acid for use according to the invention can be targeted to a particular organ or tumour location through intubation of an orifice, or with a surgical intervention.

In embodiments of the invention that relate to rectal administration the 3-hydroxydodecanoic acid for use according to the invention may be formulated for delivery as a suppository, enema or as part of an endo- or colonoscopy procedure.

The pharmaceutical compositions of the present invention can be subjected to conventional pharmaceutical operations such as sterilization and/or can contain conventional inert diluents, lubricating agents, or buffering agents, as well as adjuvants, such as preservatives, stabilizers, wetting agents, emulsifiers and buffers, etc. They may be produced by standard processes, for instance by conventional mixing, granulating, dissolving or lyophilizing processes. Many such procedures and methods for preparing pharmaceutical compositions are known in the art, see for example L. Lachman et al. The Theory and Practice of Industrial Pharmacy, 4th Ed, 2013 (ISBN 8123922892).

The invention is further illustrated by the following examples and figures, from which further embodiments and advantages can be drawn. These examples are meant to illustrate the invention.

### Description of the Figures

Time course analyses show the mean ± the standard error of the mean (SEM) analysed by two-way analysis of variance (ANOVA), with Dunnett's post-test correction for multiple comparisons. Bar graphs indicate the mean ± SEM analysed by Mann-Whitney test for comparison of two groups, or the Kruskal-Wallis test with Dunn's correction for multiple comparison. * *P*<0.05, ***P*<0.01, ****P*<0.001, *****P*<0.001.
- Fig.1: **A,** Experimental setting of 4 clostridia consortia (CC4) supplementation in a prophylactic setting in combination with anti-PD1 immunotherapy or IgG isotype control. **B,** MC-38 tumour growth curve (mm³) in mice treated with CC4 or saline plus anti-PD1 or IgG isotype control. **C,** Tumour volume (mm³) and **D** tumour weight (g) at termination. Means ± SEM analysed by Kruskal-Wallis and Dunn's correction for multiple comparison (C and D). Two-way ANOVA with Dunnett's correction for multiple comparisons (B). All experiments include five mice per group and were performed at least twice.

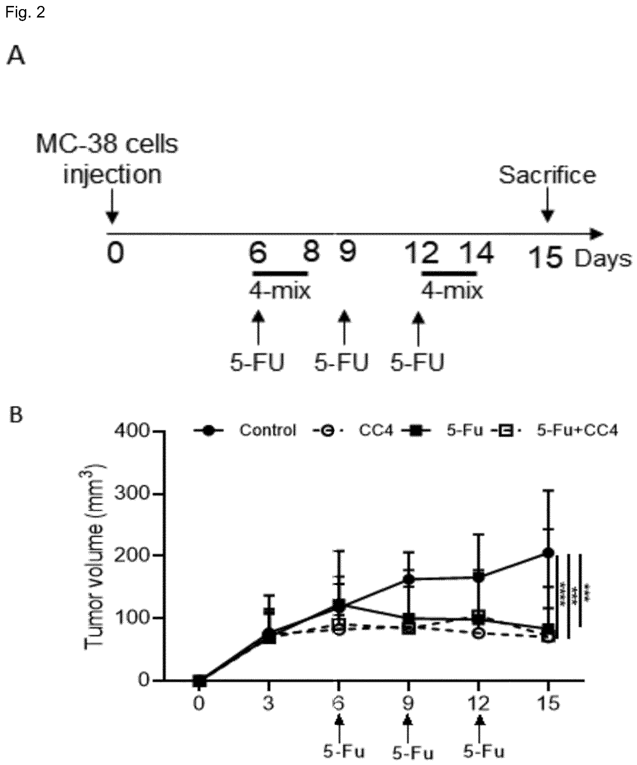

- Fig. 2: **A,** Experimental setting. **B,** MC-38 tumour volume curve (mm³) in WT untreated mice or mice treated with CC4 and/or 5-FU. Two-way ANOVA with Dunnett's correction for multiple comparisons (B). All experiments include five mice per group and were performed at least twice.

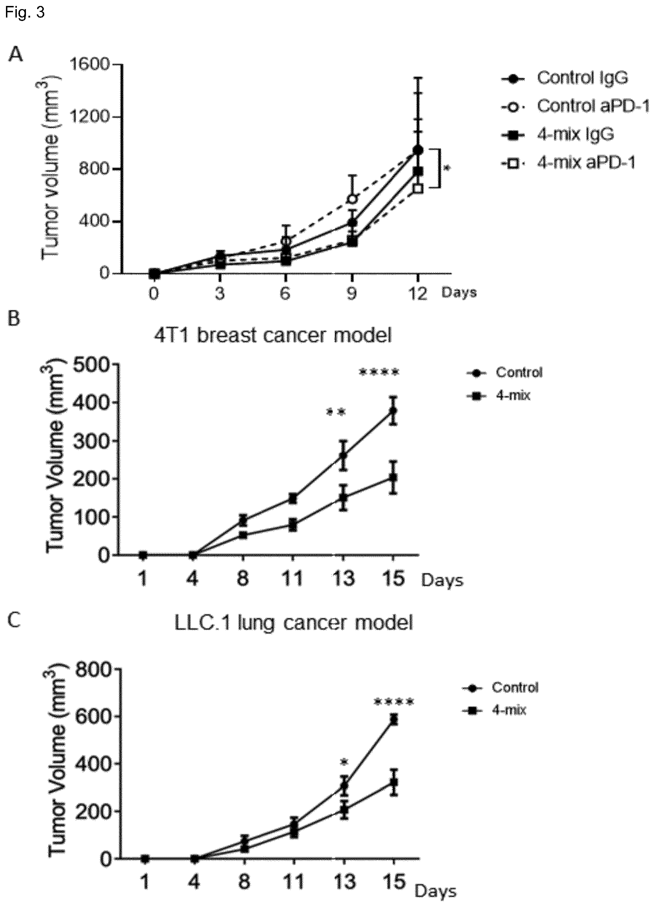

- Fig. 3: **A,** B16 melanoma tumour growth curve in control and CC4-treated mice with or without anti-PD-1 therapy. **B,** Tumour growth curve (mm³) in 4T1 breast cancer and C, LLC.1 lung cancer models. Means ± SEM analysed by Mann-Whitney test (A) or two-way ANOVA and Dunnett's correction for multiple comparison. All experiments include five mice per group and were performed at least twice.

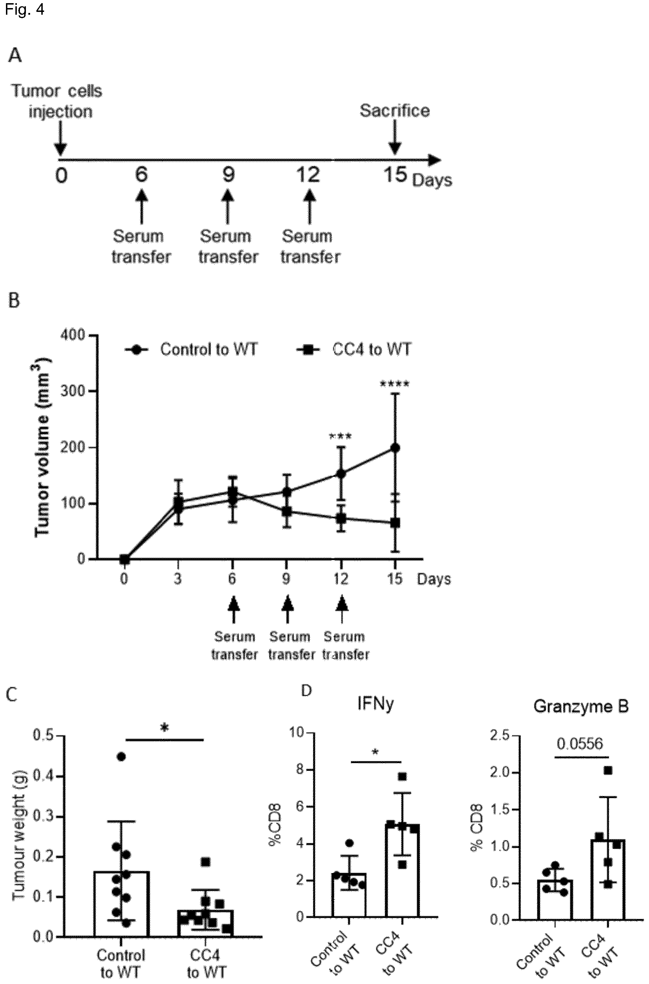

- Fig. 4: **A,** Experimental setting. **B,** MC-38 tumour growth curve (mm³) in WT mice treated with CC4-derived serum or serum from control mice. C, Tumour weight (g) in tumours from mice treated with serum from controls or CC4-treated mice. **D,** Bar charts of flow analysis in spleen corresponding to the serum transfer experiment. Means ± SEM analysed by Mann-Whitney test (C and D). Two-way ANOVA with Dunnett's correction for multiple comparisons (B). All experiments include five mice per group and were performed at least twice.

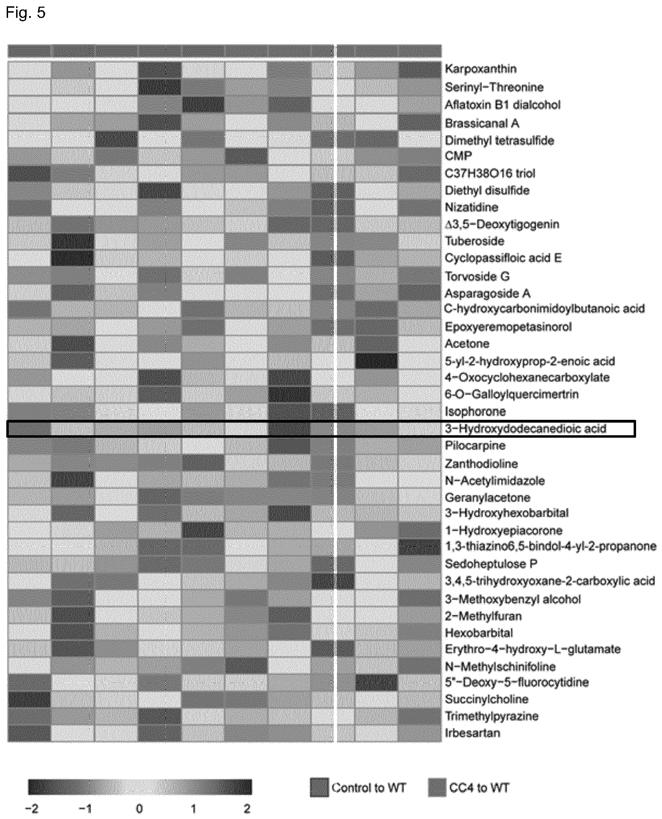

- Fig. 5: Metabolomics from serum transfer experiment. Heat-map showing the top-50 differentially expressed metabolites in WT mice treated with CC4-derived serum or serum from control mice.

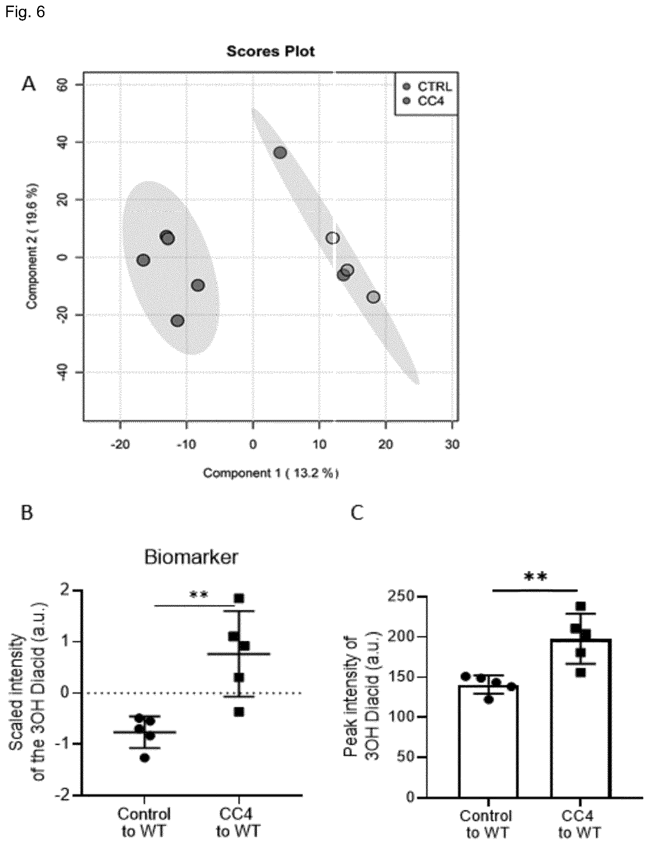

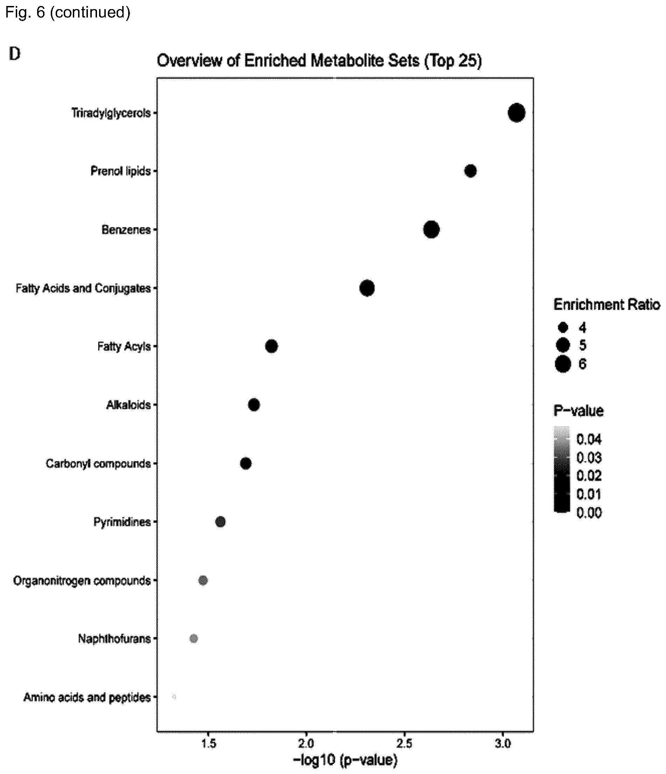

- Fig. 6: Metabolomics from serum transfer experiment. **A,** PLSDA plot showing the clustering of samples from WT mice treated with CC4-derived serum or serum from control mice. **B,** Box plot of biomarker analysis showing the feasibility of 3-OH dodecanedioic as a potential biomarker. C, Graph representing the peak intensities (a.u) of 3-OH dodecanedioic in WT mice treated with CC4-derived serum or serum from control mice. **D,** Overview of enriched metabolites sets (Top 25) showing enrichment ratio and p-value corresponding to the previous two sample groups analysed.

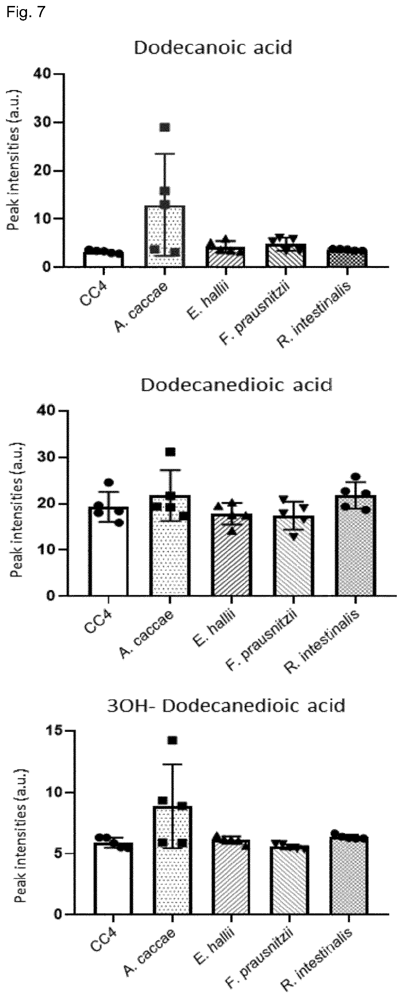

- Fig. 7: Peak intensities of dodecanoic acid, dodecanedioic acid and 3-OH dodecanedioic acid measured by mass spectrometry in bacteria supernatant (CC4, A. *caccae, E. hallii, F. prausnitzii* and *R. intestinalis*).

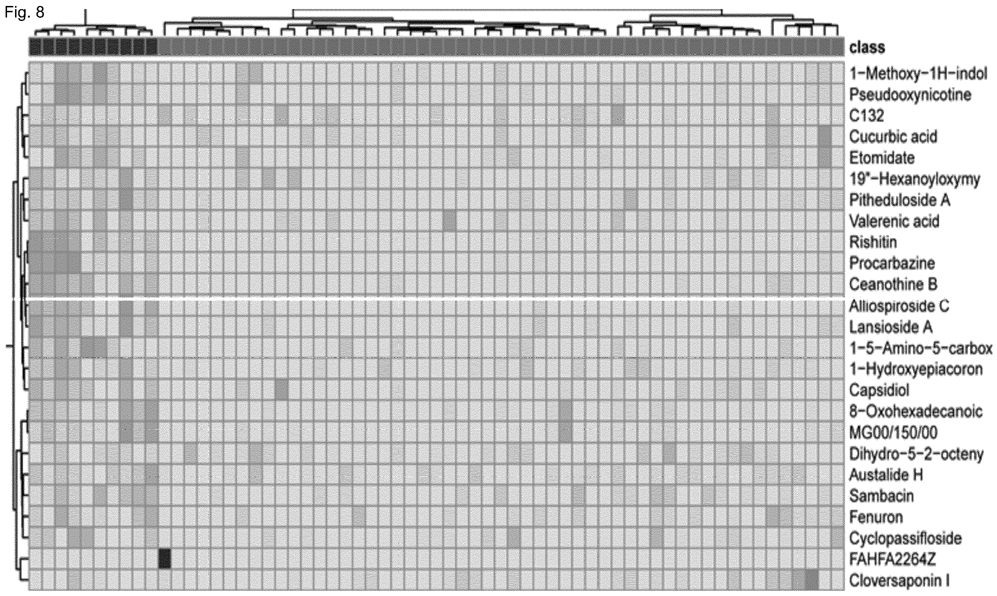

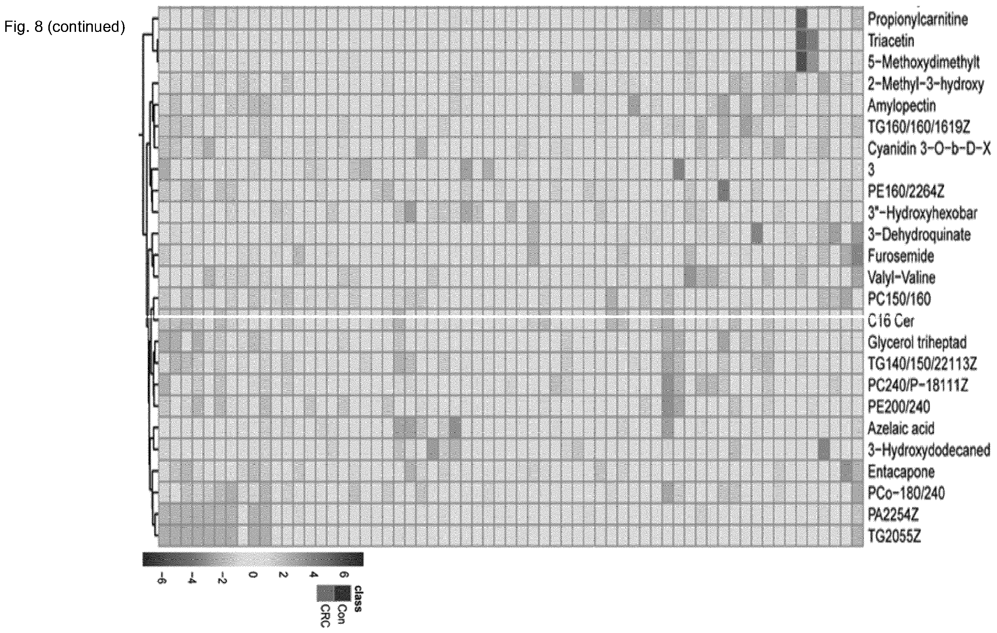

- Fig. 8: Heat-map showing the top-50 differentially detected metabolites in serum from CRC patients and healthy volunteers.

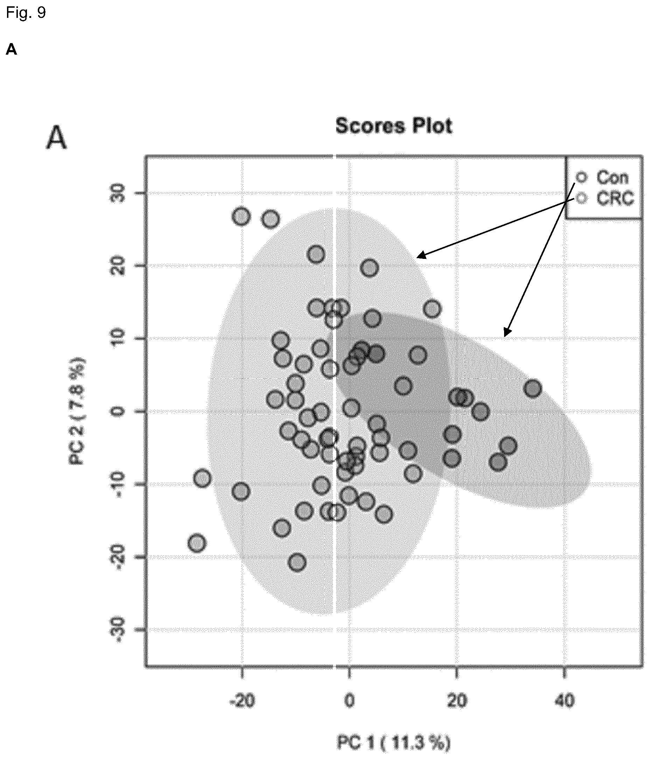

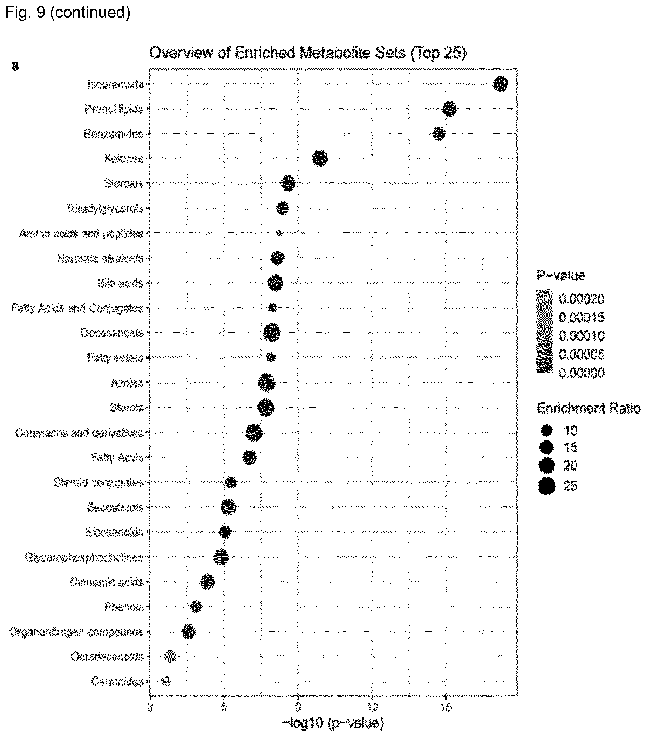

- Fig. 9: **A,** PCA plot showing the clustering of samples from healthy controls and CRC patients. **B,** Overview of enriched metabolites sets (Top 25) showing enrichment ratio and p-value corresponding to the previous two sample groups analysed.

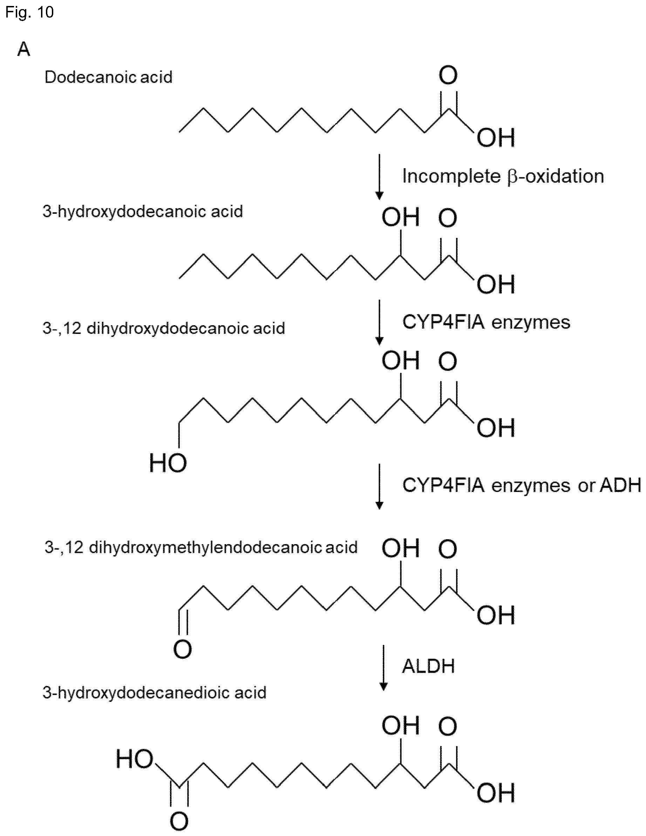

- Fig. 10: Representation of the ω-oxidation pathway, showing how fatty acids are transformed into 3-OH fatty acids (carboxylic acids) and finally into 3-OH dicarboxylic acids.

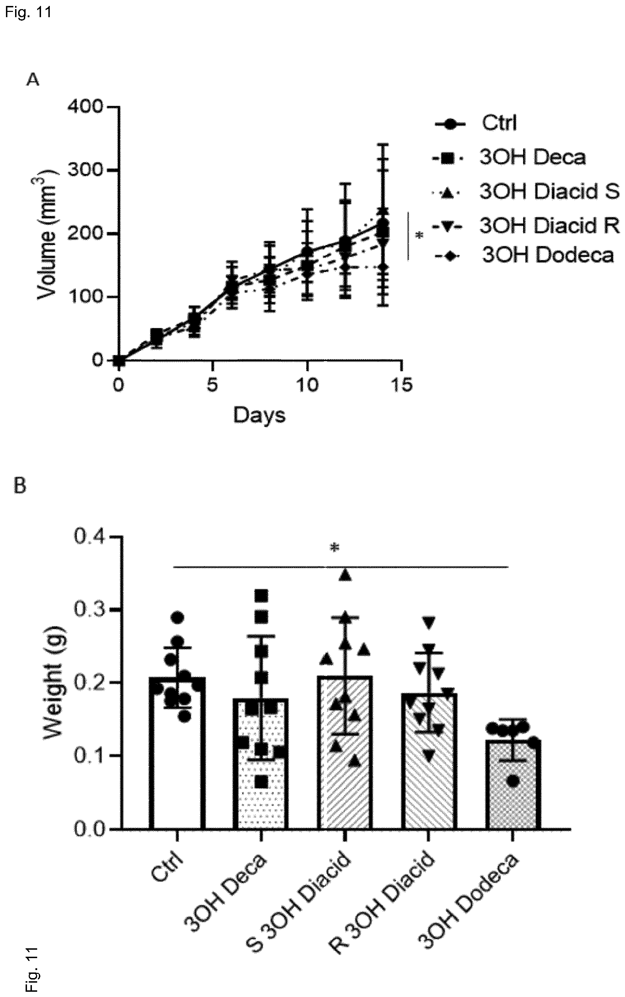

- Fig. 11: MC-38 tumour volume curve (mm³) (**A**) and tumor weight (**B**) in WT mice treated with 3-hydroxydecanoic (3OH Deca), 3-hydroxydodecanedioic acid S-enantiomer (3OH Diacid S), 3-hydroxydodecanedioic acid R- enantiomer (3OH Diacid R), 3-hydroxydodecanoic acid (3OH Dodeca) or vehicle as control (Ctrl). Means ± SEM analysed by Kruskal-Wallis and Dunn's correction for multiple comparison (B). Two-way ANOVA with Dunnett's correction for multiple comparisons (A). All experiments include five mice per group and were performed at least twice.

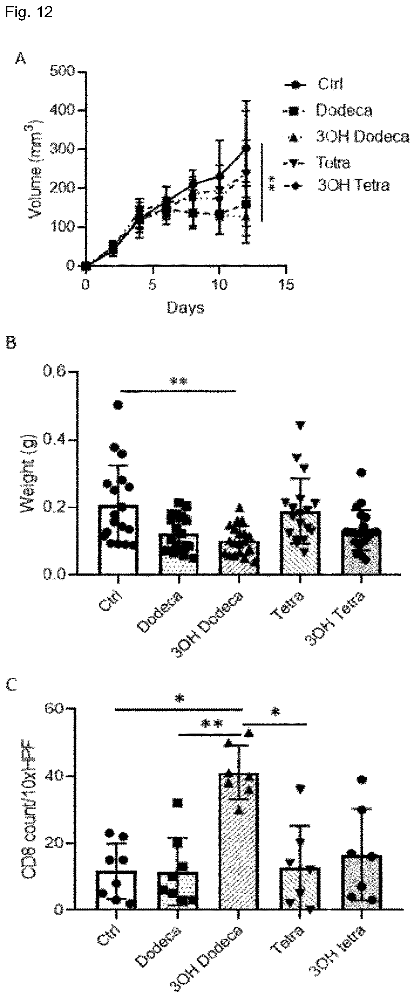

- Fig 12.: MC-38 tumour growth curve (mm³) (**A**) and tumour weight (**B**) in WT mice treated with dodecanoic acid, 3-OH dodecanoic acid, tetradecanoic acid, 3-OH tetradecanoic acid or vehicle (Ctrl). C, Quantification of CD8+ T cells (CD8+ cell/ 10xHPF) in histological slides from CD8 immunohistochemistry for the groups previously mentioned. Means ± SEM analysed by Kruskal-Wallis and Dunn's correction for multiple comparison (B and C). Two-way ANOVA with Dunnett's correction for multiple comparisons (A). **p<0.01, 2 or more pooled experiments of n=5.

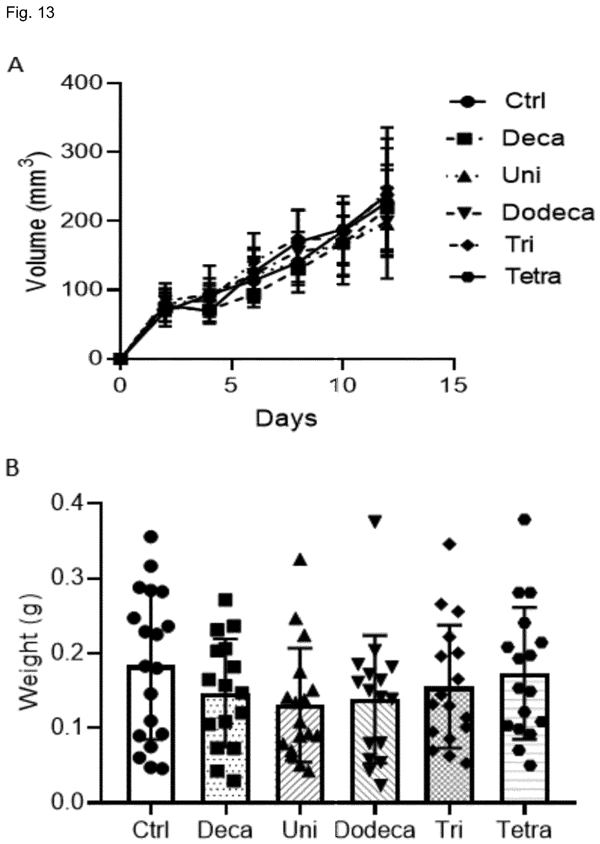

- Fig. 13.: MC-38 tumour growth curve (mm³) (**A**) and tumour weight (**B**) in WT mice treated with medium-chain fatty acids (C-10-C14). Means ± SEM analysed by Kruskal-Wallis and Dunn's correction for multiple comparison (B). Two-way ANOVA with Dunnett's correction for multiple comparisons (A). 2 or more pooled experiments of n=5-6.

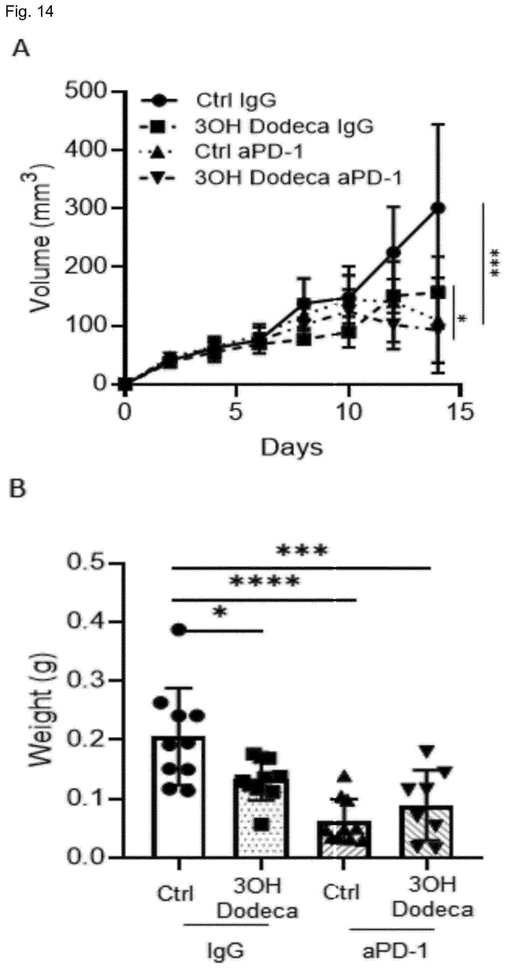

- Fig. 14.: **A,** MC-38 tumour growth curve (mm³) in mice treated with 3-OH dodecanoic (3-OH Dodeca) or saline plus anti-PD1 or IgG isotype control. **B,** Tumour weight (g) at termination. All experiments include five mice per group and were performed at least twice. Means ± SEM analysed by Kruskal-Wallis and Dunn's correction for multiple comparison (B). Two-way ANOVA with Dunnett's correction for multiple comparisons (A). *p<0.05, ***p<0.001, ****p<0.0001.

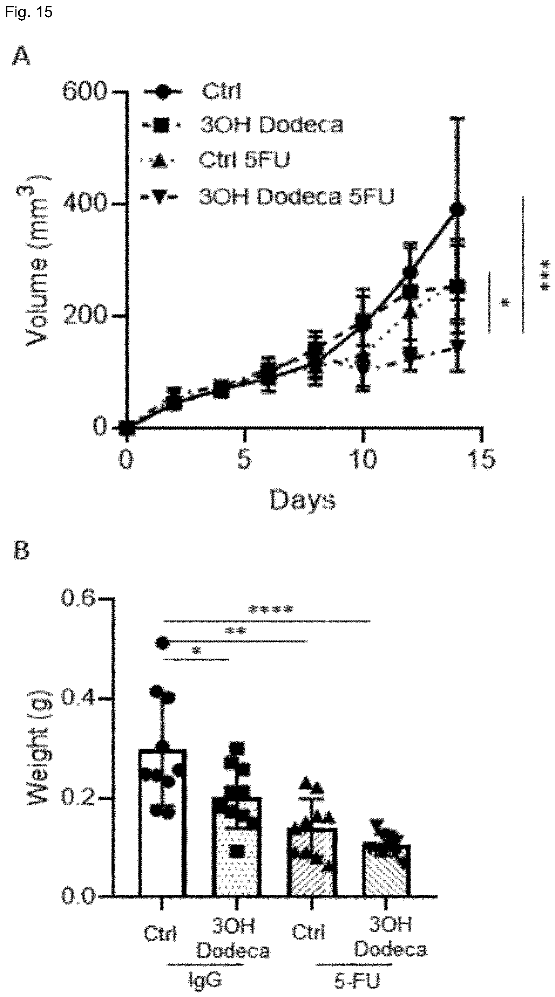

- Fig. 15.: **A,** MC-38 tumour volume curve (mm³) in WT mice treated with 3-OH dodecanoic (3-OH Dodeca) or saline plus 5-FU or vehicle. Means ± SEM analysed by Kruskal-Wallis and Dunn's correction for multiple comparison (B). Two-way ANOVA with Dunnett's correction for multiple comparisons (A). *p<0.05, **p<0.01, ***p<0.001, ****p<0.0001. 2 pooled experiments of n=5.

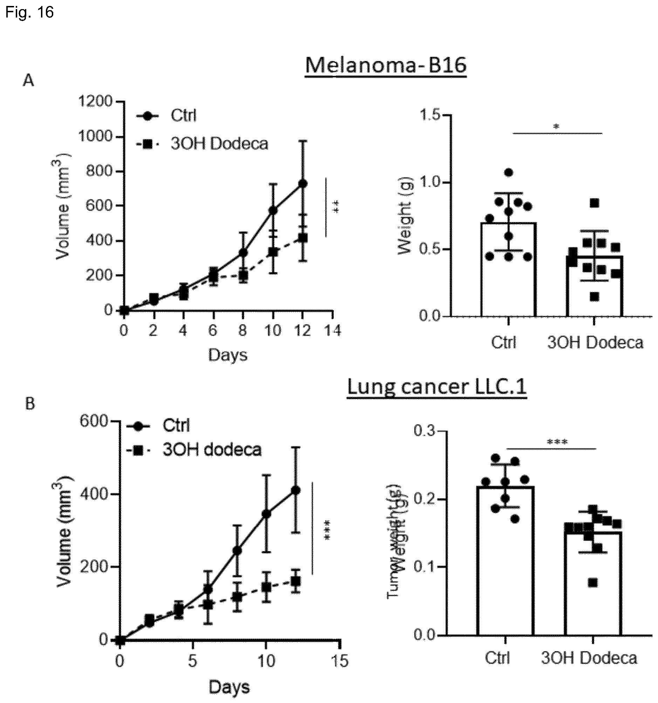

- Fig. 16: **A,** B16 tumour volume curve (mm³) and tumour weight (g) in WT mice treated with 3-OH dodecanoic (3-OH Dodeca) or saline. **B,** LLC.1 tumour volume curve (mm³) and tumour weight (g) in WT mice treated with 3-OH dodecanoic (3-OH Dodeca) or saline. Means ± SEM analysed by Kruskal-Wallis and Dunn's correction for multiple comparison (tumour weight). Two-way ANOVA with Dunnett's correction for multiple comparisons (tumour volume).

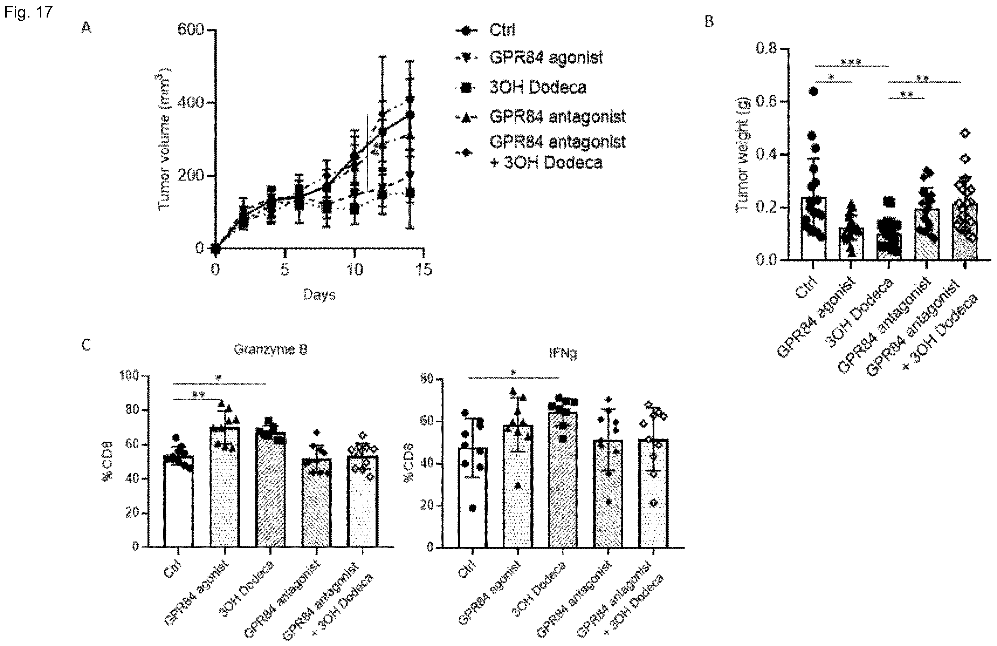

- Fig. 17.: **A,** MC-38 tumor volume curve (mm³) and **B,** tumor weight (g) in WT mice treated with GPR84 agonist, 3-OH dodecanoic acid (3-OH Dodeca), GPR84 antagonist, GPR84 antagonist & 3-OH Dodeca or saline (Ctrl). C, Bar charts of flow analysis in tumor corresponding to the groups described in **A** and **B.** Two-way ANOVA with Dunnett's correction for multiple comparisons (A & C). Means ± SEM analyzed by Kruskal-Wallis and Dunn's correction for multiple comparison (B). Experiments were performed at least twice, n=5.

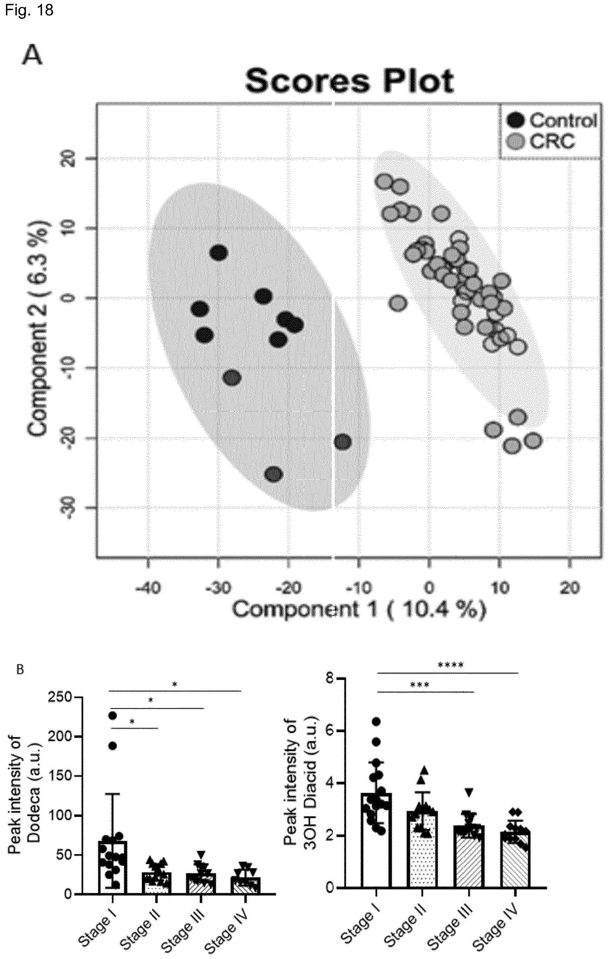

- Fig. 18.: Metabolomics of human serum samples. **A,** PLSDA plot showing the clustering of samples from control population and CRC patients. **B,** Peak intensities of dodecanoic acid (Dodeca) and 3-hydroxydodecanedioic acid (3-OH Diacid) in patients having stages I-IV of CRC. Two-way ANOVA with Dunnett's correction for multiple comparisons.

For all figures, *p<0.05, **p<0.01, ***p<0.001, ****p<0.0001.

### Examples

### Methods

### Mice

WT C57BL/6JRJ were purchased from Janvier Labs (France). All mice were kept in specific-pathogen-free conditions. Males and female littermates between 8-12 weeks were used for all the experiments. All animal experiments were performed according to Swiss animal welfare legislation and approved by the local veterinary office (Veterinäramt des Kantons Zürich).

### Bacteria culture and treatment

*Clostridiales* strains *Eubacterium hallii*: DSM3353T, *Faecalibacterium prausnitzii*: DSM17677, *Roseburia intestinalis*: DSM14610T, *Anaerostipes caccae*: DSM14662T were obtained from PharmaBiome AG, Zürich (sometimes referred to as butyrate producers, 4 clostridia consortia CC4, or BP). All strains were cultivated at 37°C in yeast extract, casitone and fatty acid (YCFA) medium (Lopez et al. 2012. Appl Environ Microbiol 78, 420-428). Strains were maintained under anoxic CO₂ atmosphere using Hungate techniques. The mix of butyrate-producing bacteria was prepared using equal volumes of two-day cultures of each bacterial strain. Purity of bacteria was confirmed by Gram stain microscopy and analysis of their metabolite production using High-Performance-Liquid-Chromatography. Viability of cells was confirmed using propidium iodide staining measured by flow cytometry. Bacteria were administered by oral gavage in a concentration of 10⁸-10⁹ live bacteria / ml in 200 microliter volume. 0.9% NaCl was used as placebo.

### Serum transfer

Serum samples were collected from mice receiving 4-mix *Clostridiales,* or placebo, in the MC38 subcutaneous cancer model. Pooled serum was transferred intraperitoneally to recipient mice bearing subcutaneous MC38 tumours at days 6, 9 and 12 post-subcutaneous injection in a volume of 200µl.

### Subcutaneous tumour models and treatments

For CRC injection models, MC38 cells were suspended in DMEM high glucose cell culture medium mixed 1:1 with Matrigel and 300,000 cells injected subcutaneously into the flanks, and mice euthanized ~3 weeks after injection. Tumour development was measured every 3 days using a digital calliper. Tumour volume was calculated using the ellipsoid formula: 4/3*3.14*Length/2* (Width/2)², where the shorter dimension was used as width and depth. Mice were terminated 14 days after subcutaneous injection or when tumour volume reached 1 cm³ or the length reached 2 cm. For the other solid tumours subcutaneous injection models, B16 melanoma cells were suspended in DMEM high glucose cell culture medium mixed 1:1 with Matrigel and 200,000 cells injected into both flanks of the animal. Lewis lung carcinoma cells LLC1.1 were suspended in DMEM high glucose cell culture medium mixed 1:1 with Matrigel and 200,000 cells injected in the right flank of a mouse. PD-1 blockade was performed in the subcutaneous tumour model by injecting of 200 µg/mouse anti-PD1(CD279) antibody (BioXCell; clone 29F.1A12) or IgG isotype control (BioXCell; clone 2a3) i.p. on days 6, 9 and 12 after subcutaneous MC-38 cells injection. 5-Fluorouracil (5-FU) was administered by i.p injections of 20 mg/kg 5-FU dissolved in DMSO at days 6, 9, and 12.

### Metabolite treatment and GPR84-targeted treatment

Each metabolite was dissolved in dimethyl sulfoxide (DMSO) to reach the stock concentration of 100mM. For mice injections, stock solution of each metabolite was diluted 1:500 in saline to reach final concentration of 200uM and 200uL/mouse were injected intraperitoneally on day 6, day 9 and day 12-post tumour cell injection. Metabolites used: 3-Hydroxydodecanedioic acid (R and S forms) (collaborators UZH), Dodecanedioic acid (Sigma), Decanoic acid (Sigma), Unidecanoic acid (Sigma), Dodecanoic acid (Sigma), Tridecanoic acid (Sigma), Tetradecanoic acid (Angene), 3-Hydroxydecanoic acid (Sigma), 3-Hydroxydodecanoic acid (Sigma), 3-Hydroxytetradecanoic acid (Angene). GPR84 receptor blockade was performed in the subcutaneous tumour model by injecting 200uL/mouse of 200uM of GPR84 antagonist GLPG1205 (MCE MedChemExpress). GPR84 activation was performed in the subcutaneous tumour model by injecting 200uL/mouse of 200uM of GPR84 agonist ZQ-16 (Sigma) i.p. on day 6, 9 and 12 after subcutaneous MC-38 cells injections.

### T cell culture

CD8+ T cells were isolated from the spleen of WT C57BL/6JRj mice and enriched using Easysep CD8+ T cells enrichment kit (Stemcell), according to the manufacturer's protocol. 100,000 CD8+ T cells were incubated in 24-well for 24h in 10% CO₂ incubator at 37°C with 10µM, 50µM, and 100µM of 3-OHTDA or media (RPMI with 10%FCS) as control. 96-well plates were either pre-coated with anti-CD3 and anti-CD28 antibodies to activate the T cells, uncoated wells were used for naïve T cells analysis.

### Human samples

Blood samples from healthy controls and CRC patients (male and female with varying age and tumour stage) before the surgery were collected at the University Hospital Zurich, Zurich, Switzerland. All samples were collected in accordance with local ethics regulations, and patients provided written informed consent prior to sample collection. Blood samples were collected to serum collection vacutainers, the blood left to clot for 15mins and clot removed by centrifuging at 1,000xg for 10mins at 4°C. Resulting serum was used for metabolomics analysis. The study was approved by the Cantonal Ethics Committee of the Canton Zürich, Switzerland (approval no. KEK-ZH-1755).

### Metabolomics

Metabolites were extracted from serum samples (20 µl) with 180 µl of methanol 80%. Samples were mixed thoroughly and vortexed for 15 seconds to precipitate protein. Samples were incubated for 1h at 4 °C, then centrifuged at RT at 14'000g for 15 min to pellet the precipitate. 100 µl of supernatant were stored at -20°C until further analysis. Metabolomics analyses were carried out with a high-resolution mass spectrometer (Agilent QTOF 6550) as described previously (Fuhrer et al. Anal. Chem. 2011, 83:18, 7074-7080, 2011). Sample was normalized by scaling to the average of the log₁₀ of the intensities of all annotated features. Significance analysis was done by heteroscedastic (two-tailed, unequal variance) t-test. In addition, p-values were adjusted according to Benjamini-Hochberg (BH), and q-values according to Storey and Tibshirani.

### Statistical analysis

When comparing two groups, non-parametric two-tailed Mann Whitney test was used. For comparisons between three or more groups, non-parametric two-tailed Kruskal-Wallis test was used and Dunn's post-hoc test applied. Correlation analyses were performed using linear regression.

### Example 1:

### Oral Clostridiales bacteria are more effective than anti-PD-1 therapy

Ineffectiveness of immune checkpoint blockade immunotherapy in CRC, and other cancers such as melanoma or bladder cancer is often due to poor T cell infiltration into the tumours. The inventors have previously found that oral administration of a clostridia consortia comprising the four *Clostridiales* strains *Eubacterium hallii*: DSM3353T, *Faecalibacterium prausnitzii*: DSM17677, *Roseburia intestinalis*: DSM14610T, *Anaerostipes caccae*: DSM14662T (CC4) ameliorates mouse models of cancer in a CD8⁺ T cell dependent manner. A therapeutic approach of anti-PD-1 treatment in combination with the CC4 consortium was tested for synergistic effects (Fig. 1A). Anti-PD-1 therapy showed only marginal anti-tumour efficacy the CRC model, as in most patients with this disease (Fig. 1B). In contrast, CC4 treatment significantly reduced tumour growth compared to anti-PD-1 treated mice, suggesting that in this model treatment with CC4 is more efficient than anti-PD-1 immunotherapy (Fig. 1B, C and D). Combination of CC4 and anti-PD-1 did not show additional anti-tumour benefit (Fig. 1B, C and D).

### Clostridiales bacteria are as efficient against CRC as the standard-of-care 5-FU

While causality is difficult to demonstrate in human data, murine models allow investigations into possible therapeutic effects of reintroducing helpful bacteria into the intestine once a tumour is established. To provide further clinical context, the efficacy of oral CC4 was compared with the clinical standard-of-care chemotherapeutic agent 5-fluorouracil (5-FU). The CC4 treatment reduced tumour growth to a similar extent as 5-FU (Fig. 2), demonstrating that CC4 is as efficient as the clinical standard of care chemotherapy with 5-FU in this context.

### Oral Clostridiales bacteria therapy inhibits the growth of tumours from diverse tissues

To determine whether oral *Clostridiales* treatment has a more universal anti-tumour capacity, the CC4 and its single bacterial strains components were tested in the B16 melanoma model, with or without combination with an anti-PD1 immunotherapy. Administration of CC4 in combination with anti-PD1 reduced the size of B16 tumours (Fig. 3A). Further tests in the 4T1 breast cancer model (Fig. 3B) and the LLC1.1 lung cancer model (Fig. 3C) found that anti-tumour efficacy of CC4 was comparable to results obtained in the CRC and melanoma models. These data identify the anti-tumour mechanism of oral bacterial treatment in both CRC and a broad range of solid tumours in both a prophylactic and therapeutic setting, showing they may be promising therapy in cancers resistant to immunotherapy treatment.

### The observed anti-tumour effect is transferable by serum injections from bacteria-treated mice

As CC4 bacterial mix and the single strains exert a systemic antitumour efficacy as shown by their effectiveness in heterotopic models, the observed anti-tumour response might be caused by circulating bacterial products. A serum-transfer strategy was used to test this hypothesis, where pooled serum from control or CC4-treated mice was injected intraperitoneally (i.p) every 72h into WT mice bearing subcutaneous MC-38 tumours (Fig. 4A). Mice receiving serum from CC4-treated mice in a therapeutic setting, exhibited a drastic tumour shrinkage over time compared to control-serum treated mice (Fig. 4B, C and D), pointing towards a soluble bacterial-derived product as the presumed key player in driving the anti-tumour immune response.

### Molecular mechanism of protection of CC4 treatment

Unbiased metabolomic profiling was performed on serum from serum-treated tumour-bearing mice (experiment corresponding to figure 4), revealing metabolites that differed between the serum or mice receiving control, or CC4 treatment-derived serum. An increased level of 3-hydroxydodecanedioic acid was observed among the 50 most-differentially detected metabolites in the serum of mice treated with CC4-derived serum (CC4) compared to those mice treated with serum from control mice (Ctrl) (Fig. 5). Principle component analysis identified a clear grouping corresponding to the two different treatment groups, animals receiving serum from control mice (Ctrl) and those receiving the serum from CC4-treated mice (CC4) (Fig. 6A). In a biomarker analysis, 3-hydroxydodecanedioic acid (3OH diacid) presented an area under the curve (AUC) value close to 1, indicating a strong rationale for use as a biomarker (Fig. 6B). Peak intensities also demonstrated a higher level of 3-hydroxydodecanedioic acid (3OH diacid) in CC4 treated mouse serum compared to control mice (Fig. 6C). Moreover, a metabolite enrichment analysis showed an abundance of the fatty acids and conjugates within the groups analysed (Fig. 6D)

### 3-hydroxydodecanedioic acid is a bacterial-derived metabolite

To investigate the origin of the identified metabolite 3-hydroxydodecanedioic acid, further metabolomics analysis was performed on the bacterial supernatant of cultured CC4 and the corresponding single strains (A. *caccae, E. hallii, F. prausnitzii* and *R. intestinalis*), assessing production of 3-hydroxydodecanedioic acid and its derivates, including the fatty acids dodecanoic acid and dodecanedioic acid. Although fatty acids were not abundantly expressed in most of the bacterial compositions with the exception of *A. caccae,* dodecanedioic acid and 3-hydroxydodecanedioic acid peak intensities indicated comparable production by all single strains and CC4 (Fig. 7).

### 3-hydroxydodecanedioic acid is detectable in human serum

After identifying 3-hydroxydodecanedioic acid as potential biomarker in mouse serum, metabolite profiling was then performed on serum acquired from human CRC patients prior to surgical intervention (CRC), and from healthy volunteers (Con). A heatmap identified 3-hydroxydodecanedioic acid within the top 50 of the most differentially expressed metabolites between the groups analysed (Fig. 8). A principal component analysis showed that clusters of distinct metabolites distinguished patients from healthy controls (Fig. 9A). As with bacterial culture supernatants, enrichment analysis showed the group of fatty acids and conjugates within the top-10 of most enriched metabolites in the human setting (Fig. 9B).

### 3-hydroxydodecanoic acid has a potent anti-tumour effect in vivo

The ω-oxidation pathway characteristic of the bacterial metabolism (Fig. 10), generates 3-hydroxy fatty acid (carboxylic acid) intermediates during the production of 3-hydroxydicarboxylic acids. To determine which molecules in this pathway might underlie the observed anti-tumour effect, mice were treated with 3-hydroxydodecanoic acid (3OH Dodeca), two enantiomers of 3-hydroxydodecanedioic acid (3OH Diacid S and 3OH Diacid R), 3-hydroxydecanoic acid (3OH Deca) as an additional carboxylic acid, and compared to a vehicle alone group as a control (Ctrl). 3-hydroxydodecanoic acid demonstrated the most favourable anti-tumour effect in terms of reduced tumour volume (Fig. 11 A) and weight (Fig. 11 B).

### 3-hydroxydodecanoic acid carbon chain length and hydroxylation

As 3-hydroxydodecanoic and not 3-hydroxydodecanedioic acid demonstrated the strongest anti-tumour effect in vivo, the importance of the 3-hydroxyl group was assessed. Mice were treated with dodecanoic acid, 3-hydroxydodecanoic acid, tetradecanoic acid, 3-hydroxytetradecanoic acid, or vehicle as control. In terms of both tumour volume (Fig. 12 A) and tumour weight (Fig. 12 B) the anti-tumour effect of the bacterial metabolites was specifically induced by 3-hydroxydodecanoic acid. A similar experiment examined a broader spectrum of medium-chain fatty acids (C10-C14), however, none of the medium-chain fatty acids had an anti-tumour effect compared to control group (Fig. 13 A and B).

### 3-hydroxydodecanoic acid has a similar anti-tumour effect to anti-PD1 or 5-FU

In order to examine the efficacy of metabolite treatment according to current standard of care treatments, mice were treated with either 3-hydroxydodecanoic acid, anti-PD-1 or the combination of both, in addition to an untreated control group. A comparable anti-tumour effect was observed between 3-hydroxydodecanoic acid and anti-PD-1, and the combination of both treatments did not show any further beneficial effect in either tumour volume (Fig. 14 A) or tumour weight (Fig. 14 B). Similarly, the effect of 3-hydroxydodecanoic acid was compared with the chemotherapeutic 5-fluorouracil (5-FU), the combination of both, and an untreated control group. Again, a comparable anti-tumour effect between 3-hydroxydodecanoic acid and 5-FU was observed, and the combination of both treatments did not show any further beneficial effect in either tumour volume (Fig. 15 A) or tumour weight (Fig. 15 B). 3-hydroxydodecanoic acid, a naturally occurring metabolite in the gut mucosal environment, is not known to be associated with toxicity or side effects, in contrast to these known chemotherapeutic and checkpoint inhibitor agents.

### 3-hydroxydodecanoic acid is effective in a broad number of tumour models

Finally, additional solid tumour experiments were carried out to determine if the anti-tumour effect 3-hydroxydodecanoic acid was relevant to non-gastrointestinal tumours. Mouse subcutaneous injection models of both melanoma (B16) and lung cancer (LLC.1) were treated mice with 3-hydroxydodecanoic acid or vehicle as control. As previously seen for the CRC model, both B16 (Fig. 16 A) and LLC.1 (Fig. 16 B) models showed a clear anti-tumour effect upon administration of 3-hydroxydodecanoic acid.

### Anti-tumour effect of 3-hydroxydodecanoic acid is dependent on GPR84 receptor signalling

To understand whether an interaction with the medium chain fatty acid sensitive G protein-coupled receptor 84 is involved in the molecular mechanism of 3-hydroxydodecanoic acid, an experiment with a GPR84-specific agonist and antagonist was carried out. Activation of GPR84 receptor by a synthetic agonist 2-(hexylthio)pyramidine-4,6-diol, a lipid-mimetic structure comprising a pyrimidine ring (Zhang Q. et al. 2016 J. Pharmacol. And Exp. Therpeut. May 337-344), resulted in similar tumour reduction comparing to 3-hydroxydodecanoic acid, while GPR84 blockade completely abrogated the anti-tumour effect of 3-hydroxydodecanoic acid (Fig. 17A-B). Treatment with GPR84 agonist and 3-hydroxydodecanoic acid both resulted in the similarly increased expression of granzyme B and IFNy by CD8+ T cells, while mice which had GPR84 receptor blocked and received 3-hydroxydodecanoic acid entirely lost this anti-tumour immunological change (Fig. 17C).

### 3-hydroxydodecanoic acid and 3-hydroxydodecanedioic acid are reduced in stage III and IV CRC tumours in human

PCA analysis was undertaken on metabolic profiles obtained for samples from CRC patients and healthy subjects, and metabolites form differentiating clusters were examined. The metabolic analysis revealed high abundance of dodecanoic acid (Dodeca) and 3-hydroxydodecanedioic acid revealed levels in the stage I tumours with the gradual decrease of both metabolites in advanced CRC stages (n=15 stage I, n=14 stage II, n=11 stage III and n=9 stage IV)(Fig. 18B), such that decreasing concentration of dodecanoic acid or its derivative are strongly associated with progression to late stage colon cancer.

## Claims

1. A hydroxycarboxylic acid of the formula
(I) CH₃(CH₂)₈ CHOH-(CH₂)-CO₂H,
or a pharmaceutically acceptable salt thereof,
for use in the treatment or prevention, including prevention of recurrence, of cancer.

2. A pharmaceutical formulation of the hydroxycarboxylic acid or the pharmaceutically acceptable salt thereof as specified in claim 1, formulated for enteral, topical, intra-tumoural, intravenous and/or parenteral administration for use in the treatment or prevention, including prevention of recurrence, of cancer.

3. The hydroxycarboxylic acid or the pharmaceutically acceptable salt thereof for use according to claim 1, or the pharmaceutical formulation for use according to claim 2, wherein the cancer is colorectal cancer.

4. The hydroxycarboxylic acid, pharmaceutically acceptable salt thereof, or the pharmaceutical formulation for use as specified in any one of the claims 1 to 3, wherein the cancer is a solid malignant tumour **characterized by** a decrease or absence of tumour-infiltrating immune cells.

5. The hydroxycarboxylic acid, pharmaceutically acceptable salt thereof, or the pharmaceutical formulation for use, as specified in any one of the claims 1 to 4, wherein the cancer is a solid malignant tumour **characterized by** an absence of tumour-infiltrating CD8+ T cells.

6. A combination medicament for use in the treatment or the prevention of recurrence of cancer comprising the hydroxycarboxylic acid or the pharmaceutically acceptable salt thereof, or the pharmaceutical formulation for use as specified in any one of the claims 1 to 5, and an antineoplastic pharmaceutical drug.

7. The combination medicament for use according to claim 6, wherein the antineoplastic pharmaceutical drug is a checkpoint inhibitor antibody, particularly a checkpoint inhibitor antibody selected from an anti-PD-1, anti-PD-L1, anti-PD-L2, or anti-CTLA-4 antibody.

8. The hydroxycarboxylic acid, the pharmaceutically acceptable salt thereof, or the pharmaceutical formulation for use as specified in any one of the claims 1 to 5, wherein the hydroxycarboxylic acid or pharmaceutical composition is administered to a patient without concurrent, previous, or subsequent administration of an additional antineoplastic pharmaceutical drug.

9. The hydroxycarboxylic acid, pharmaceutically acceptable salt thereof, or the pharmaceutical formulation for use as specified in any one of the claims 1 to 5, for use in a patient without concurrent, previous, or subsequent administration of a checkpoint inhibitor antibody, or a chemotherapeutic agent.

## Patentansprüche

1. Eine Hydroxycarbonsäure der Formel
(I) CH₃(CH₂)₈ CHOH-(CH₂)-CO₂H,
oder ein pharmazeutisch geeignetes Salz davon,
zur Verwendung bei der Behandlung oder Vorbeugung, einschließlich der Vorbeugung eines Wiederauftretens, von Krebs.

2. Eine pharmazeutische Formulierung der Hydroxycarbonsäure oder des pharmazeutisch geeigneten Salzes davon wie in Anspruch 1 beschrieben, formuliert zur enteralen, topischen, intratumoralen, intravenösen und/oder parenteralen Verabreichung zur Verwendung bei der Behandlung oder Vorbeugung, einschließlich der Vorbeugung eines Wiederauftretens, von Krebs.

3. Die Hydroxycarbonsäure oder das pharmazeutisch geeignete Salz davon zur Verwendung gemäß Anspruch 1 oder die pharmazeutische Formulierung zur Verwendung gemäß Anspruch 2, wobei es sich bei dem Krebs um Darmkrebs handelt.

4. Die Hydroxycarbonsäure, das pharmazeutisch geeignete Salz davon oder die pharmazeutische Formulierung zur Verwendung wie in einem der Ansprüche 1 bis 3 beschrieben, wobei der Krebs ein solider bösartiger Tumor ist, der durch eine Abnahme oder das Fehlen von tumorinfiltrierenden Immunzellen gekennzeichnet ist.

5. Die Hydroxycarbonsäure, das pharmazeutisch geeignete Salz davon oder die pharmazeutische Formulierung zur Verwendung, wie in einem der Ansprüche 1 bis 4 beschrieben, wobei der Krebs ein solider bösartiger Tumor ist, der durch das Fehlen von tumorinfiltrierenden CD8+-T-Zellen gekennzeichnet ist.

6. Ein Kombinationsarzneimittel zur Verwendung bei der Behandlung oder Vorbeugung des Wiederauftretens von Krebs, umfassend die Hydroxycarbonsäure oder das pharmazeutisch geeignete Salz davon oder die pharmazeutische Formulierung zur Verwendung wie in einem der Ansprüche 1 bis 5 beschrieben und ein antineoplastisches Arzneimittel.

7. Das Kombinationsarzneimittel zur Verwendung gemäß Anspruch 6, wobei das antineoplastische Arzneimittel ein Checkpoint-Inhibitor-Antikörper ist, insbesondere ein Checkpoint-Inhibitor-Antikörper, ausgewählt aus einem Anti-PD-1-, Anti-PD-L1-, Anti-PD-L2- oder Anti-CTLA-4-Antikörper.

8. Die Hydroxycarbonsäure, das pharmazeutisch geeignete Salz davon oder die pharmazeutische Formulierung zur Verwendung wie in einem der Ansprüche 1 bis 5 beschrieben, wobei die Hydroxycarbonsäure oder die pharmazeutische Formulierung einem Patienten ohne gleichzeitige, vorherige oder nachfolgende Verabreichung eines zusätzlichen antineoplastischen Arzneimittels verabreicht wird.

9. Die Hydroxycarbonsäure, das pharmazeutisch geeignete Salz davon oder die pharmazeutische Formulierung zur Verwendung wie in einem der Ansprüche 1 bis 5 beschrieben zur Verwendung bei einem Patienten ohne gleichzeitige, vorherige oder nachfolgende Verabreichung eines Checkpoint-Inhibitor-Antikörpers oder eines Chemotherapeutikums.

## Revendications

1. Acide hydroxycarboxylique de la formule
(I) CH₃(CH₂)₈ CHOH-(CH₂)-CO₂H,
ou sel pharmaceutiquement acceptable de celui-ci,
destiné à être utilisé dans le traitement ou la prévention, y compris la prévention de récurrence, du cancer.

2. Formulation pharmaceutique de l'acide hydroxycarboxylique ou du sel pharmaceutiquement acceptable de celui-ci selon la revendication 1, formulée pour l'administration par voie entérale, topique, intra-tumorale, intraveineuse et/ou parentérale destinée à être utilisée dans le traitement ou la prévention, y compris la prévention de récurrence, du cancer.

3. Acide hydroxycarboxylique ou sel pharmaceutiquement acceptable de celui-ci destiné à être utilisé selon la revendication 1, ou formulation pharmaceutique destinée à être utilisée selon la revendication 2, dans lequel/laquelle le cancer est un cancer colorectal.

4. Acide hydroxycarboxylique, sel pharmaceutiquement acceptable de celui-ci ou formulation pharmaceutique destiné(e) à être utilisé(e) selon l'une quelconque des revendications 1 à 3, dans lequel/laquelle le cancer est une tumeur maligne solide **caractérisée par** une diminution ou absence de cellules immunitaires infiltrant la tumeur.

5. Acide hydroxycarboxylique, sel pharmaceutiquement acceptable de celui-ci ou formulation pharmaceutique destiné(e) à être utilisé(e) selon l'une quelconque des revendications 1 à 4, dans lequel/laquelle le cancer est une tumeur maligne solide **caractérisée par** une absence de lymphocytes T CD8+ infiltrant la tumeur.

6. Médicament combiné destiné à être utilisé dans le traitement ou la prévention de récurrence du cancer comprenant l'acide hydroxycarboxylique ou le sel pharmaceutiquement acceptable de celui-ci ou la formulation pharmaceutique destiné(e) à être utilisé(e) selon l'une quelconque des revendications 1 à 5, et un médicament pharmaceutique antinéoplasique.

7. Médicament combiné destiné à être utilisé selon la revendication 6, dans lequel le médicament pharmaceutique antinéoplasique est un anticorps à inhibiteur de point de contrôle, notamment un anticorps à inhibiteur de point de contrôle sélectionné parmi un anticorps anti-PD-1, anti-PD-L1, anti-PD-L2 ou anti-CTLA-4.

8. Acide hydroxycarboxylique, sel pharmaceutiquement acceptable de celui-ci ou formulation pharmaceutique destiné(e) à être utilisé(e) selon l'une quelconque des revendications 1 à 5, dans lequel/laquelle l'acide hydroxycarboxylique ou la composition pharmaceutique est administré(e) à un patient sans administration concomitante, antérieure ou subséquente d'un médicament pharmaceutique antinéoplasique supplémentaire.

9. Acide hydroxycarboxylique, sel pharmaceutiquement acceptable de celui-ci ou formulation pharmaceutique destiné(e) à être utilisé(e) selon l'une quelconque des revendications 1 à 5, destiné(e) à être utilisé(e) chez un patient sans administration concomitante, antérieure ou subséquente d'un anticorps à inhibiteur de point de contrôle ou d'un agent chimiothérapeutique.
